# EUROPEAN PATENT APPLICATION

(11) **EP 4 821 894 A1**
(43) Date of publication of application: **14.10.2026**
(21) Application number: 25169162.2
(22) Date of filing: 08.04.2025
(51) Int. Cl.: C02F 1/36, A61L 2/025, C02F 103/32, C02F 103/42

(54) **WATER TREATMENT SYSTEM USING ULTRASONIC WAVES WITH ENHANCED WAVE DIFFUSION**

(71) Applicant: Lagur A/S, 8550 Ryomgård (DK)
(72) Inventor: Brøns Pedersen, Kim, 8550 Ryomgård (DK)
(74) Representative: Høiberg P/S

(57) **Abstract**

The invention regards a system for treatment of water, comprising a treatment enclosure (2) comprising an inlet (3) and an outlet (4) for introducing a water stream through the treatment enclosure, and an ultrasonic transducer (5), wherein the ultrasonic transducer is arranged to generate ultrasonic waves inside the treatment enclosure, and wherein the treatment enclosure comprises at least one scattering element (6) arranged to reflect the ultrasonic waves in a plurality of different angles thereby diffusing the ultrasonic waves in the treatment enclosure.

## Description

### Technical field

The present disclosure relates to a system and method for treatment of water using ultrasonic waves.

### Background

Ultrasonic treatment of water is a technique that utilizes high-frequency sound waves to induce physical and chemical effects within a water medium. These effects can include cavitation, localized heating, and mechanical agitation, which may contribute to the disruption of microorganisms or degradation of contaminants.

Conventional ultrasonic treatment systems often rely on direct transmission of ultrasonic waves from a transducer into the water. However, the distribution of acoustic energy within the treatment chamber may be uneven, with energy concentrated along certain paths and diminished in others. This may limit the overall effectiveness of treatment, as certain regions within the water volume may be insufficiently exposed to ultrasonic effects.

There remains a need for an ultrasonic water treatment system that promotes more uniform energy distribution within the treatment volume without significantly increasing structural or operational complexity.

### Summary

Considering the prior art described above, it is an object of the present disclosure to provide a system for treatment of water using ultrasonic waves. The system can provide enhanced distribution of the ultrasonic waves in the volume of water that is treated.

The ultrasonic waves induce cavitation inside the treatment enclosure. Cavitation refers to the formation, growth, and implosive collapse of microscopic gas or vapor bubbles in a liquid when subjected to rapid changes in pressure, such as those generated by high-frequency ultrasonic waves. When the local pressure in the liquid falls below the vapor pressure, small cavities or bubbles form. As the pressure recovers, these bubbles collapse violently, releasing localized energy in the form of heat, shock waves, and high shear forces.

Cavitation can be harnessed to produce mechanical and chemical effects that contribute to the disruption of contaminants and microorganisms. The intense forces generated during bubble collapse can damage or rupture bacterial cell walls, leading to inactivation or death of microbial organisms.

The present disclosure relates to a system for treatment of water, comprising
- a treatment enclosure comprising an inlet and an outlet for introducing a water stream through the treatment enclosure, and
- an ultrasonic transducer,

wherein the ultrasonic transducer is arranged to generate ultrasonic waves inside the treatment enclosure, and
wherein the treatment enclosure comprises at least one scattering element arranged to reflect the ultrasonic waves in a plurality of different angles thereby diffusing the ultrasonic waves in the treatment enclosure.

The system may provide several advantages related to the effectiveness and efficiency of ultrasonic water treatment. By incorporating at least one scattering element within the treatment enclosure, ultrasonic waves generated by the ultrasonic transducer can be reflected at multiple angles, leading to a more uniform distribution of acoustic energy throughout the treatment enclosure. This diffusion of ultrasonic waves can enhance interaction between the waves and contaminants or microorganisms in the water stream, potentially increasing the likelihood of bacterial disruption or removal. The arrangement may also reduce the formation of acoustic dead zones, where little or no ultrasonic energy would otherwise reach, thereby improving overall treatment consistency.

The system can be used for treatment of potable water, such as drinking water, in residential and commercial buildings, where the goal is to improve hygiene and reduce microbial content without chemical additives.

The system can be used for treatment of hot water in domestic systems, where conditions may support the growth of bacteria such as legionella.

Treatment of process water in light industrial or food and beverage settings can be performed using the system of the present disclosure, where non-chemical disinfection may be preferred to avoid product contamination.

The system can be used for treatment of pool and spa water, where continuous flow treatment can support microbial control in recirculating systems.

The system can be used for treatment of irrigation water in controlled environments such as greenhouses, where reducing biofilm or algae buildup in pipes and nozzles may improve water delivery and crop health or for treatment of rainwater used in non-potable systems, where microbial load reduction enhances safety for secondary uses.

In one embodiment, the treatment enclosure comprises rigid walls with a thickness of at least 0.5 cm, preferably in the range of 0.5 cm and 2 cm, such as in the range of 0.8 cm and 1.5 cm.

Providing the treatment enclosure with rigid walls with thickness in the specified range may improve the structural stability and durability of the system during operation. The use of relatively thick walls may reduce deformation or vibration caused by the ultrasonic waves, which can contribute to more efficient transmission of acoustic energy into the water. This configuration may also help to confine the ultrasonic energy within the enclosure, minimizing energy loss through the walls and enhancing the overall intensity of the treatment process. Increased wall thickness could further offer greater resistance to mechanical wear.

In one embodiment, the walls of the treatment enclosure are made of a metal or metal alloy, preferably from stainless steel. Constructing the walls of the treatment enclosure from a metal or metal alloy, such as stainless steel, may offer several advantages in terms of mechanical strength, thermal stability, and resistance to corrosion. Metallic materials are well-suited to withstand the stresses generated by ultrasonic vibrations and internal water pressure, supporting consistent system performance over time. Stainless steel, in particular, may provide a hygienic and chemically resistant surface that is compatible with a wide range of water qualities and treatment environments.

In one embodiment, the at least one scattering element is made from the same material as the walls of the treatment enclosure. Forming the scattering element from the same material as the walls of the treatment enclosure may simplify manufacturing and ensure material compatibility within the system. This approach can provide a uniform mechanical and thermal response across the enclosure, reducing the risk of differential expansion, detachment, or material fatigue under operating conditions and supporting effective reflection and diffusion of ultrasonic waves. In addition, selecting a corrosion-resistant and durable material for both components may contribute to the overall longevity and reliability of the system, especially in continuous or demanding water treatment applications.

In one embodiment, the ultrasonic transducer is attached to an outer side of one of the walls of the treatment enclosure, and wherein a barrier is provided between the ultrasonic transducer and an inside of the treatment enclosure of a thickness in the range between 0.1 cm and 0.5 cm, such as between 0.15 cm and 0.3 cm, preferably 0.16 cm.

The ultrasonic transducer is attached on the outer side of a wall of the treatment enclosure which allows the transducer to transmit energy into the enclosure without direct contact with the water stream. This may reduce the risk of fouling, corrosion, or contamination. The presence of a barrier of controlled thickness between the transducer and the interior of the enclosure may support efficient acoustic coupling while maintaining physical separation. A barrier within the specified thickness range may strike a balance between mechanical strength and acoustic transparency, allowing effective transmission of ultrasonic waves into the water while preserving the integrity of the enclosure. This configuration may also simplify maintenance, such as replacement of the transducer, without interrupting the sealed environment of the treatment enclosure.

In one embodiment, the barrier is formed integrally in one of the walls of the treatment enclosure. This may reduce the number of separate components and potential sealing interfaces in the system, thereby simplifying assembly and lowering the risk of leakage. A barrier formed integrally in one of the walls of the treatment enclosure can enhance mechanical stability and ensure consistent acoustic transmission by eliminating gaps or material discontinuities between the transducer mounting surface and the interior of the enclosure.

In one embodiment, the treatment enclosure has a volume in the range between 200 cm³ and 1000cm³, such as between 400 cm³ and 600 cm³. A controlled enclosure volume may help ensure that the ultrasonic energy is distributed efficiently throughout the water stream, increasing the likelihood of uniform treatment. Volumes between the specified range may support a balance between sufficient residence time for the water and high throughput capacity, making the system suitable for integration into existing piping or flow systems without requiring significant space or infrastructure modifications.

In one embodiment, the system is connected to a water pipe with nominal diameter in the range between 20 mm and 60 mm, such as between 25 mm and 40 mm. Nominal diameter is commonly used to describe the size of a pipe without specifying the outer or inner diameter of the pipe. Connection to a water pipe with nominal diameter in the specified range may allow for compatibility with a wide range of commonly used pipe sizes in residential, commercial, and industrial water systems. This range accommodates standard plumbing dimensions, facilitating integration into existing installations without the need for extensive modifications. This compatibility may enhance the versatility of the system and broaden its potential use across different applications and infrastructure types.

In one embodiment, the ultrasonic transducer has a frequency between 30 kHz and 100 kHz, preferably between 40 kHz and 80 kHz. Using an ultrasonic transducer with a frequency within the specified range may enable the system to generate cavitation effects suitable for disrupting microorganisms and promoting effective water treatment. Frequencies within this range are commonly associated with strong cavitation activity, providing a balance between intensity and control of bubble dynamics. Operating at these frequencies may enhance the mechanical effects on bacteria and particles in the water while minimizing the risk of damage to system components.

In one embodiment, the ultrasonic transducer has an output power between 30 W and 100 W, such as between 50 W and 80 W, preferably of 60 W. An ultrasonic transducer with an output power within the specified range, may provide sufficient energy to generate effective cavitation and acoustic streaming within the treatment enclosure. This power range may support reliable disruption of microorganisms and enhance mixing and contact between the ultrasonic waves and the water stream. The specified range may also reduce the risk of overheating or wear on system components, contributing to long-term performance and stability.

In one embodiment, the at least one scattering element is positioned on the inner surface of the wall opposite the ultrasonic transducer and in a propagation direction of the ultrasonic waves.

Positioning the scattering element on the inner surface of the wall opposite the ultrasonic transducer and in a propagation direction of the ultrasonic waves may enhance the distribution of acoustic energy within the treatment enclosure. This placement allows the incoming ultrasonic waves to interact directly with the scattering element after traversing the water volume, promoting reflection at multiple angles and reducing the formation of standing waves or areas with low acoustic intensity. By redirecting the waves back into the enclosure in a diffused manner, this configuration may improve overall energy uniformity and increase the likelihood that all regions of the water stream are exposed to cavitation effects, thereby supporting more consistent and effective treatment.

In alternative embodiments, one or more scattering elements are provided as one or more bars extending through the treatment enclosure of the system between opposing walls and crossing the propagation direction of the ultrasonic waves. The bars may e.g. have a triangular, rectangular or a circular cross-section.

In one embodiment, the wall of the treatment enclosure opposite the ultrasonic transducer and in a propagation direction of the ultrasonic waves comprises a curved inner surface constituting a scattering element.

A curved inner surface on the wall opposite the ultrasonic transducer and in a propagation direction of the ultrasonic waves may function as an effective scattering element by altering the reflection angles of incoming waves. This geometry can help to break up directional wave patterns and redistribute the acoustic energy more evenly throughout the treatment enclosure. Compared to flat surfaces, a curved surface may reduce the likelihood of wave focusing or the creation of acoustic dead zones, supporting a more homogeneous treatment environment.

In one embodiment, the wall opposite the ultrasonic transducer and in a propagation direction of the ultrasonic waves is parallel to the direction of the water stream. This may support efficient flow dynamics while maintaining optimal acoustic interaction within the treatment enclosure. This alignment allows the ultrasonic waves to travel across the water stream rather than directly along it, increasing the exposure of the moving fluid to the acoustic field. As the water flows past the wavefront, more volume may be subjected to consistent cavitation and mixing effects. Additionally, maintaining parallel orientation may help reduce flow disturbances or pressure drops, allowing the system to integrate smoothly into existing piping configurations without adversely affecting hydraulic performance.

In one embodiment, the at least one scattering element is a conical, hemispherical, tetrahedral, quadrilateral pyramid, frustum or irregular structure.

Using a scattering element of one of the above mentioned shapes may enhance the diffusion of ultrasonic waves within the treatment enclosure by reflecting the waves in multiple directions. These geometries introduce varying surface angles and contours that can disrupt uniform wave paths, promoting more even distribution of acoustic energy and minimizing the formation of standing waves or untreated zones. The variety of shapes allows for flexibility in design, enabling the scattering effect to be tailored to the enclosure's geometry and the specific acoustic characteristics of the system. Irregular or multi-faceted surfaces could create a complex and dynamic ultrasonic field, which can improve cavitation coverage and contribute to more thorough and reliable treatment of the water stream.

The present disclosure further relates to the use of the system according to the present disclosure for reducing and/or eliminating bacteria, such as legionella bacteria, in the water stream.

The use of the system for reducing and/or eliminating bacteria, such as legionella bacteria, in the water stream may provide a non-chemical and energy-efficient approach to microbial control. By applying ultrasonic waves that induce cavitation and related mechanical effects, the system may disrupt bacterial cell structures and biofilms, leading to a reduction in bacterial presence without the need for chemical disinfectants or high-temperature treatment. This approach may be particularly beneficial in applications where chemical-free operation is preferred or where traditional disinfection methods are ineffective or impractical. The ability to target bacteria such as legionella may support improved water hygiene and safety in environments including domestic water systems, cooling towers, and healthcare or hospitality facilities.

Legionella bacteria are a group of waterborne pathogens, with Legionella pneumophila being the species most commonly associated with human disease. These bacteria are naturally found in freshwater environments but can become a health concern when they proliferate in man-made water systems such as hot water tanks, plumbing systems, cooling towers, and decorative fountains. Legionella thrive in warm water, particularly in temperatures between 20 °C and 50 °C, and can form resilient biofilms on surfaces inside pipes and reservoirs. Inhalation of aerosolized water droplets containing Legionella can lead to Legionnaires' disease, a severe form of pneumonia, or Pontiac fever, a milder flu-like illness. The bacteria are particularly dangerous for individuals with weakened immune systems, the elderly, or those with underlying respiratory conditions.

Controlling Legionella typically requires rigorous maintenance of water systems, including the use of chemical disinfectants (e.g., chlorine, monochloramine), thermal disinfection, or physical interventions. However, these methods can have drawbacks such as chemical by-products, material corrosion, or high energy consumption. Therefore, a system that can reduce or eliminate Legionella using ultrasonic cavitation offers a promising alternative. The mechanical disruption of bacterial cells and biofilms through ultrasound may limit bacterial growth and persistence without the disadvantages associated with conventional treatment methods, supporting safer and more sustainable water management.

The present disclosure further relates to the use of the system according to the present disclosure for reducing or preventing formation of limescale in pipes and equipment connected downstream of the system.

The present disclosure further describes a method for treating of water via ultrasonic waves. The method comprising the steps of
- providing a treatment enclosure comprising an inlet and an outlet for introducing a water stream through the treatment enclosure,
- providing an ultrasonic transducer,
- generating ultrasonic waves inside the treatment enclosure by means of the ultrasonic transducer,
- diffusing the ultrasonic waves in the treatment enclosure by means of an at least one scattering element arranged to reflect the ultrasonic waves in a plurality of different angles, and
- treating the water with diffused ultrasonic waves generated by the ultrasonic transduces and diffused by means of the at least one scattering element while the water flows though the treatment enclosure.

The described method may offer efficient and uniform treatment of water by enhancing the distribution of ultrasonic energy within the treatment enclosure. By diffusing the ultrasonic waves through a scattering element, the method may increase the exposure of the water stream to cavitation effects, improving the disruption of microorganisms and contaminants. The continuous flow configuration allows for real-time treatment without interrupting the water supply, making the method suitable for integration into existing systems. Additionally, the use of ultrasonic waves provides a chemical-free approach to water treatment, supporting environmentally friendly and low-maintenance operation.

### Description of the drawings

The disclosure will in the following be described in greater detail with reference to the accompanying drawings:
Fig. 1 a schematic side view of a system for treatment of water according to the present disclosure
Fig. 1A zoomed in area of Fig. 1 showing the thickness of the walls and barrier of the system according to embodiments of the present disclosure
Fig. 2 a schematic side view of a system for treatment of water attached to a water pipe
Fig. 3 a schematic side view of a system for treatment of water comprising a scattering element, illustrating the diffusion of the ultrasonic waves in the treatment enclosure
Fig. 4-5 a schematic side view of a system for treatment of water comprising a curved inner wall that constitutes a scattering element
Fig. 6-9 a schematic side view of a system for treatment of water, showing different embodiments of the scattering element

### Detailed description

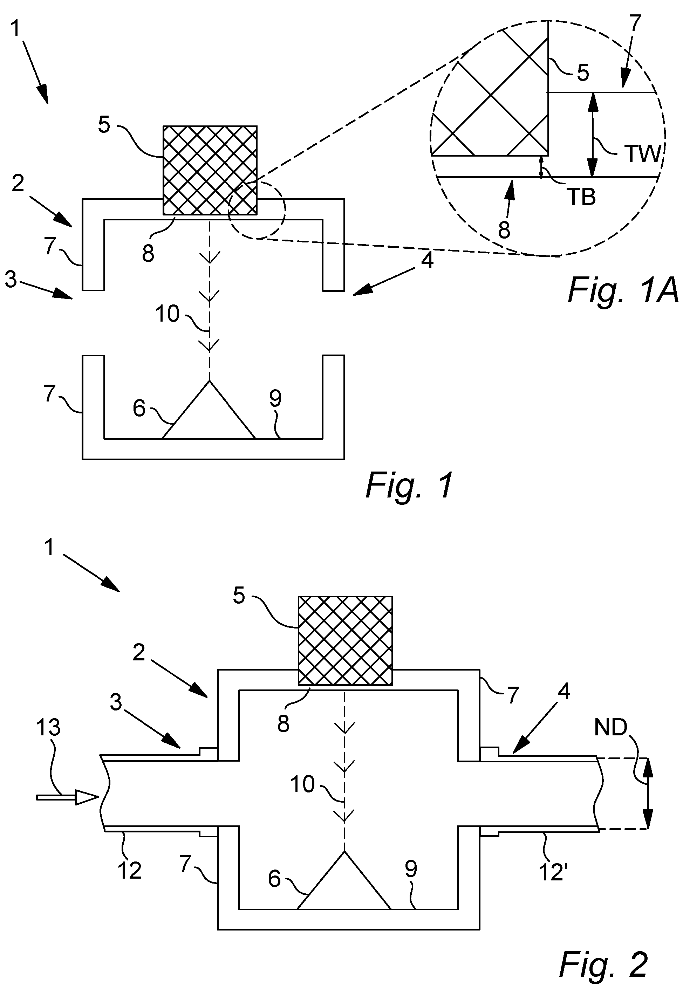

Fig. 1 shows a side view of a system 1 for treatment of water. The system 1 comprises a treatment enclosure 2 and an ultrasonic transducer 5.

The treatment enclosure 2 comprises an inlet 3 and an outlet 4 for introducing a water stream 13 (not shown in the figure) through the treatment enclosure 2.

The ultrasonic transducer 5 is arranged to generate ultrasonic waves inside the treatment enclosure 2.

The treatment enclosure 2 comprises at least one scattering element 6 arranged to reflect the ultrasonic waves in a plurality of different angles thereby diffusing the ultrasonic waves in the treatment enclosure 2.

The treatment enclosure 2 comprises rigid walls 7. As shown in Fig. 1A, the rigid walls 7 have a thickness TW of at least 0.5 cm. The thickness TW of the walls 7 should be at least 0.5 cm so that the walls are rigid. This is important for ensuring that the ultrasonic waves are reflected and not absorbed by the walls 7 of the treatment enclosure 2. The thickness TW of the walls 7 can preferably be in the range of 0.5 cm and 2 cm, such as in the range of 0.8 cm and 1.5 cm.

The thickness TW of the walls 7 is also important for sound attenuation. As high frequency sounds are generated by the ultrasonic transducer 5, the rigid walls 7 of the treatment enclosure 2 ensure damping of the sound. If additional sound attenuation is desired, the system 1 can be covered with a Styrofoam shell.

The ultrasonic transducer 5 does not directly contact the water within the treatment enclosure 2. In Fig. 1, the ultrasonic transducer 5 is attached to an outer side of one of the walls 7 of the treatment enclosure 2. A barrier 8 is provided between the ultrasonic transducer 5 and an inside of the treatment enclosure of a thickness TB in the range between 0.1 cm and 0.5 cm, such as between 0.15 cm and 0.3 cm, preferably 0.16 cm. A small thickness TB of the barrier 8 compared to the thickness TW of the walls 7 is desired. This is illustrated in Fig. 1A. A barrier 8 with the preferred thickness TB of 0.16 cm is advantageous as it allows for propagation of the ultrasonic waves from the ultrasonic transducer 5 to the inside of the treatment enclosure 2.

The walls 7 of the treatment enclosure 2 can be made of a metal or metal alloy, preferably from stainless steel. Stainless steel is commonly used in water pipes and water treatment devices and is an advantageous option as it is suitable for long-term contact with water, due to its corrosion resistance and durability.

The at least one scattering element 6 is made from the same material as the walls 7 of the treatment enclosure 2, which is preferably stainless steel. The use of the same material is desired so that the ultrasonic waves are reflected at the surface of the walls 7 and the surface of the scattering element 6 in the same way, e.g. no difference in absorption of the ultrasonic waves is expected.

As shown in Fig. 1, the barrier 8 can be formed integrally in one of the walls 7 of the treatment enclosure 2. This could simplify manufacturing and assembly processes. Moreover, forming the barrier 8 integrally in one of the walls can ensure that the ultrasonic waves are propagated towards the inside the treatment enclosure 2, without losses due to possible gaps between the barrier 8 and the walls 7 of the treatment enclosure 2.

Fig. 2 shows a side view of a system 1 for treatment of water that is connected to water pipes 12 and 12'. A water pipe 12 is connected to the inlet 3 and another water pipe 12' is connected to the outlet 4. A water stream 13 is introduced as the water flows from left to right in the figure. The water from the water pipe 12 passes through inlet 3, to the treatment enclosure 2 and gets treated as it flows through the treatment enclosure 2. The water exits the treatment enclosure through outlet 4 and flows through pipe 12'.

The design of the system 1 for treatment of water enables the use of the system 1 directly on a water supply, as the system 1 can be directly connected to the water pipes 12, 12'. The system 1 for treatment of water could be mounted on the water supply of residential buildings or large building complexes. The system 1 for treatment of water could also be suitable for use in swimming pools or water parks. The system 1 for treatment of water could also be suitable for use in commercial, and industrial water systems.

The treatment enclosure 2 has a volume in the range between 200 cm³ and 1000cm³, such as between 400 cm³ and 600 cm³. This volume is suitable for treating the water as it flows through the treatment enclosure 2, while the ultrasonic transducer 5 generates ultrasonic waves that are diffused by the scattering element 6.

The treatment enclosure 2 can have a shape of a cube. In this case the cube could have a preferred inner edge length of 8 cm. Alternatively, the treatment enclosure 2 can have a shape of a cuboid. In this case the cuboid could have a preferred length, width and height that is between 7 cm and 9 cm. The treatment enclosure 2 could also have a cylindrical shape with a preferred height between 7 cm and 9 cm and a preferred radius between 4 cm and 5 cm. The cylinder would be symmetrical along the propagation direction 10 of the ultrasonic waves and the ultrasonic transducer 5 and scattering element 6 would be placed each on a base of the cylinder. The side view of the system 1 for treatment of water in Fig. 2 can be considered to illustrate all shape options described here.

The system is connected to a water pipe 12, 12' with nominal diameter ND in the range between 20 mm and 60 mm, such as between 25 mm and 40 mm. The nominal diameter ND is a commonly used parameter for water pipes which, as shown in Fig. 2, does not correspond to the inner or outer diameter of the water pipe 12, 12'. The above mentioned pipe sizes are commonly used in water supply systems, which ensures that the system 1 for treatment of water is compatible and can be used directly in different water supply systems.

Another commonly use notation for pipe sizes in the nominal pipe size (NPS). Pipes 12, 12' with nominal diameter ND in the range between 20 mm and 60 mm correspond to nominal pipe size NPS ¾ to 2 ⅓ inches. Pipes 12, 12' with nominal diameter ND in the range between 25 mm and 40 mm correspond to nominal pipe size NPS 1 to 1 ½ inches.

The ultrasonic transducer 5 has a frequency between 30 kHz and 100 kHz, preferably between 40 kHz and 80 kHz. A frequency within this range, particularly around 60 kHz, could be particularly useful for inducing cavitation for the purposes of eliminating bacteria or removing algae from the water stream 13.

The ultrasonic transducer 5 has an output power between 30 W and 100 W, such as between 50 W and 80 W, preferably of 60 W. The system 1 for treatment of water can be powered by directly connecting the ultrasonic transducer 5 to a power outlet. The system 1 for treatment of water could be suitable for use in different power outlets, such as the commonly used power outlets with 230V (50 Hz) or with 115V (60 Hz). The system 1 for treatment of water is designed for continuous operation. The use of an additional battery and a converter could be advantageous for ensuring operation of the system 1 under power outage.

For energy saving purposes, the system could be on stand-by mode when there is no flow of water through the treatment enclosure. The stand-by mode, where the operation of the ultrasonic transducer 5 is stopped, could be triggered after a certain period of time with absence of flow through the treatment enclosure. For household use, this trigger could for example be enabled at night, where longer time periods without water usage are expected.

The ultrasonic transducer 5 has an output power preferably of 60 W. After propagation through the barrier 8, the power of the ultrasonic waves, is deposited on the stream of water 13, inducing cavitation and treating the water as it flows through the treatment enclosure 2.

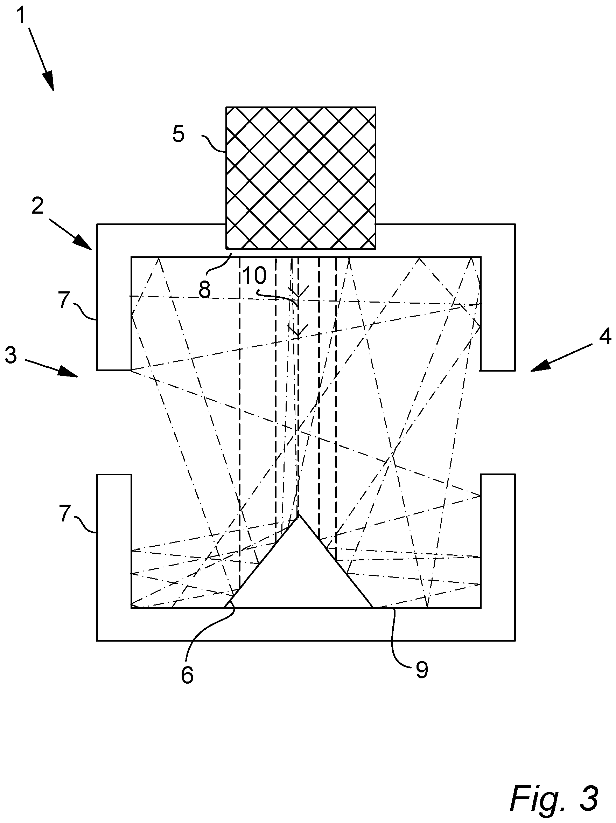

An example of the reflections that the ultrasonic waves are undergoing inside the treatment enclosure 2 is shown in Fig. 3. This is shown for ultrasonic waves propagating along the propagation direction 10 at different locations within the treatment enclosure 2. The initial ultrasonic waves propagating through the treatment enclosure 2 are shown with dashed lines, while the reflected waves are shown with dash-dotted lines.

The ultrasonic waves induce cavitation within the treatment enclosure 2. As the water is subjected to rapid changes in pressure and when the local pressure in the water falls below the vapor pressure, small cavities or bubbles form. As the pressure recovers, these bubbles collapse violently, releasing localized energy in the form of heat, shock waves, and high shear forces. The use of one or more scattering elements 6 is advantageous as it improves the diffusion of the ultrasonic waves within the treatment enclosure 2. Thus, the ultrasonic waves will release their energy in larger volume within the treatment enclosure 2, providing a more effective treatment of the water.

In Fig. 3, the scattering element 6 provides a surface for reflection of the ultrasonic waves that leads to reflection at multiple different angles. This is particularly advantageous, compared to the reflection of the ultrasonic waves only on the inner surface 9 of the wall 7, if the wall is not curved.

In all figures, the at least one scattering element 6 is positioned on the inner surface 9 of the wall 7 opposite the ultrasonic transducer 5 and in a propagation direction 10 of the ultrasonic waves. This is particularly useful as the ultrasonic waves, before being reflected at the surface of the scattering element 6, are mainly oriented along the propagation direction 10, while after reflection they are directed at multiple different angles.

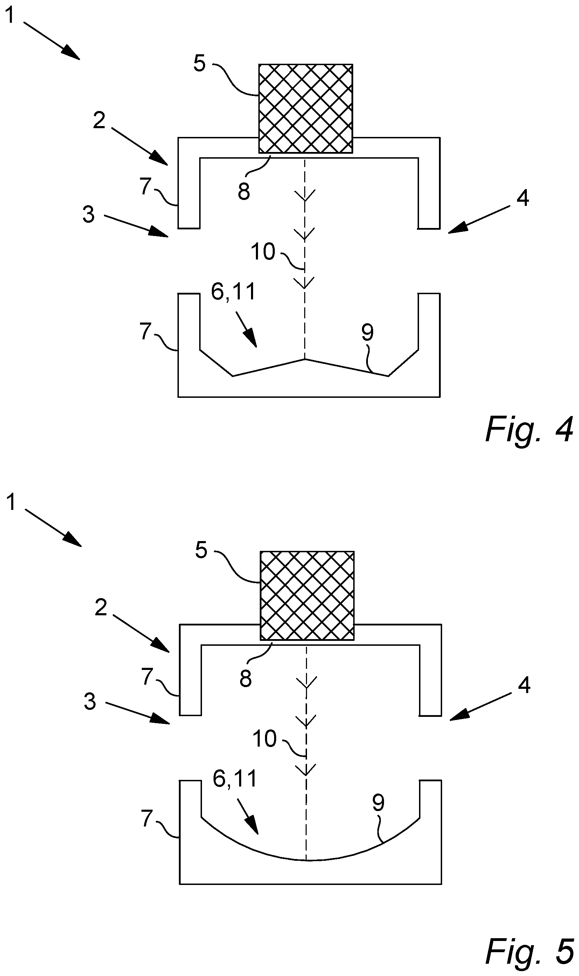

The scattering element 6 can be an element that is attached to the inner surface 9 of a wall 7 of the treatment enclosure 2 or can be formed from a wall 7. In Figs. 4-5, the wall 7 opposite the ultrasonic transducer 5 and in a propagation direction 10 of the ultrasonic waves comprises a curved 11 inner surface 9 constituting a scattering element 6. In that case, the scattering element 6 is an integral part of the wall 7 which could be beneficial for manufacturing purposes.

The curved 11 inner surface 9, for example shown in Figs. 4-5, can be combined with one or more scattering elements 6 that are positioned on the inner surface 9 of the wall 7. This combination could be advantageous as in that case, the whole area of the inner surface 9 of the wall 7 opposite the ultrasonic transducer 5 and in a propagation direction 10 of the ultrasonic waves will contribute to reflection in multiple different angles.

**The** wall 7 opposite the ultrasonic transducer 5 and in a propagation direction 10 of the ultrasonic waves is parallel to the direction of the water stream 13. That way, the water stream get treated with the ultrasonic waves at multiples positions as the water passes through the treatment enclosure 2. Additionally, the effective volume where the water gets treated is increased by implementing one or more scattering elements 6.

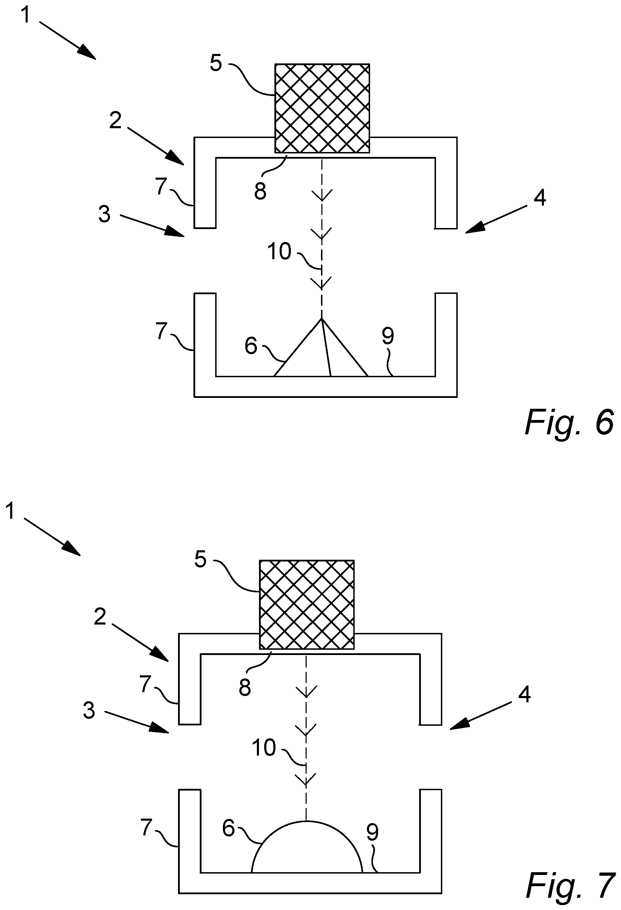

**The** at least one scattering element 6 can be constructed in different shapes. Figs. 6-9 show different examples of scattering elements with different shapes.

In Figs. 1-3, the scattering element 6 has a conical shape and is placed opposite the ultrasonic transducer 5. **The** use of a conical shape is advantageous as it provides the necessary diffusion by reflection at different angles and due to its shape can diffuse the ultrasonic waves in all directions within the volume of the treatment enclosure 2. A conical shape may be particularly effective in cylindrical enclosures or where centralized diffusion of energy is desired.

In Fig. 6, the scattering element 6 is depicted as a tetrahedral or quadrilateral pyramid. This shape can alternatively be used and could enhance the diffusion of the ultrasonic waves. A tetrahedral or quadrilateral pyramid shape, with its multiple flat faces and sharp angles, can create complex reflection patterns and break up wave coherence. This may enhance turbulent mixing and increase the randomness of wave paths, which is useful for disrupting biofilms or microbial clusters. These shapes may be advantageous in rectangular or box-shaped enclosures where strategic wave redirection is needed.

In Fig. 7, the scattering element 6 is depicted as hemispherical structure. A hemispherical surface can reflect incoming waves in a wide range of directions due to its continuous curvature. This shape may help minimize standing wave formation and provide uniform acoustic coverage, especially in enclosed or confined volumes.

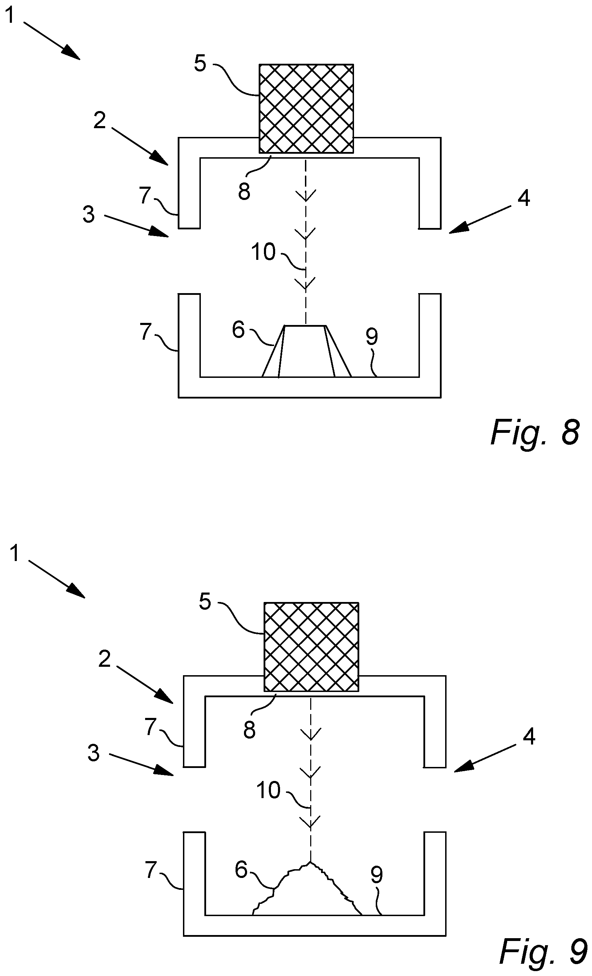

In Fig. 8, the scattering element 6 is depicted as a frustum. A frustum shape, typically a cone or pyramid with its top truncated, can reflect waves at angles that differ from those of full cones or pyramids. This allows for tailored scattering behaviour that balances wave redirection with minimal obstruction to water flow. This shape can be beneficial when the reflection along the propagation direction 10 is desired in the central area of the volume of the treatment enclosure 2, while reflection at different angles is desired at the edges of the treatment enclosure 2.

In Fig. 9, the scattering element 6 is depicted as an irregular structure. An irregular shape can be beneficial in some cases as it offers a scattering element with multiple surfaces and orientations that would create a complex diffusion of the ultrasonic waves inside the treatment enclosure 2.

The shape of the scattering element 6 and possible combination with a scattering element formed by a curved 11 inner surface 9 (not shown in figures) would depend on the size and shape of the treatment enclosure 2. The size of the scattering element 6 would also depend on the size of the treatment enclosure 2. It would be preferable that the scattering element 6 does not create a major disturbance on the stream of water 13 as it passes through the treatment enclosure 2.

The system 1 for treatment of water can be used for reducing and/or eliminating bacteria, such as legionella bacteria, in the water stream. As already described, the system 1 for treatment of water can offer an more efficient treatment of water, by diffusing the ultrasonic waves in the treatment enclosure 2. Operation of the system 1 with the specified power output of the ultrasonic transducer 5 and the specified frequency of the ultrasonic waves can lead to reduction and/or elimination of bacteria in the water stream 13. Particularly, reduction and/or elimination of legionella bacteria can be achieved when the water is treated by the system 1 for water treatment.

The system 1 for water treatment offers a chemical-free treatment of legionella bacteria. The system 1 for water treatment is designed to withstand operation for an extending period of time, which additionally does not require any maintenance. This increases the shelf life of the system 1 and eliminates additional costs for regular maintenance, offering a cost-effective solution for water treatment.

The operation of the system, in particular with the specified power output of the ultrasonic transducer 5 and the specified frequency of the ultrasonic waves, is furthermore beneficial for preventing formation of limescale in pipes and equipment connected downstream of the system 1.

### Reference list:

- 1: system for treatment of water
- 2: treatment enclosure
- 3: inlet
- 4: outlet
- 5: ultrasonic transducer
- 6: scattering element
- 7: wall
- 8: barrier
- 9: inner surface of wall
- 10: propagation direction of the ultrasonic waves
- 11: curved inner surface
- 12, 12': water pipe
- 13: stream of water
- TW: thickness of wall
- TB: thickness of barrier
- ND: nominal diameter

## Claims

1. A system (1) for treatment of water, comprising
- a treatment enclosure (2) comprising an inlet (3) and an outlet (4) for introducing a water stream (13) through the treatment enclosure (2), and
- an ultrasonic transducer (5),
wherein the ultrasonic transducer (5) is arranged to generate ultrasonic waves inside the treatment enclosure (2), and
wherein the treatment enclosure (2) comprises at least one scattering element (6) arranged to reflect the ultrasonic waves in a plurality of different angles thereby diffusing the ultrasonic waves in the treatment enclosure (2).

2. The system (1) of claim 1, wherein the treatment enclosure (2) comprises rigid walls (7) with a thickness (TW) of at least 0.5 cm, preferably in the range of 0.5 cm and 2 cm, such as in the range of 0.8 cm and 1.5 cm.

3. The system (1) according to claim 2, wherein the walls (7) of the treatment enclosure (2) are made of a metal or metal alloy, preferably from stainless steel.

4. The system (1) according to any of the preceding claims, wherein the at least one scattering element (6) is made from the same material as the walls (7) of the treatment enclosure (2).

5. The system (1) according to any of the preceding claims, wherein the ultrasonic transducer (5) is attached to an outer side of one of the walls (7) of the treatment enclosure (2), and wherein a barrier (8) is provided between the ultrasonic transducer (5) and an inside of the treatment enclosure (2) of a thickness (TB) in the range between 0.1 cm and 0.5 cm, such as between 0.15 cm and 0.3 cm, preferably 0.16 cm.

6. The system (1) according to claim (5), wherein the barrier (8) is formed integrally in one of the walls (7) of the treatment enclosure (2).

7. The system (1) according to any of the preceding claims, wherein the treatment enclosure (2) has a volume in the range between 200 cm³ and 1000cm³, such as between 400 cm³ and 600 cm³.

8. The system (1) according to any of the preceding claims, wherein the system 1 is connected to a water pipe (12,12') with nominal diameter (ND) in the range between 20 mm and 60 mm, such as between 25 mm and 40 mm.

9. The system (1) according to any of the preceding claims, wherein the ultrasonic transducer (5) has a frequency between 30 kHz and 100 kHz, preferably between 40 kHz and 80 kHz.

10. The system (1) according to any of the preceding claims, wherein the ultrasonic transducer (5) has an output power between 30 Wand 100 W, such as between 50 W and 80 W, preferably of 60 W.

11. The system (1) according to any of the preceding claims, wherein the at least one scattering element (6) is positioned on the inner surface (9) of the wall (7) opposite the ultrasonic transducer (5) and in a propagation direction (10) of the ultrasonic waves.

12. The system (1) according to any of the preceding claims, wherein the wall (7) of the treatment enclosure (2) opposite the ultrasonic transducer (5) and in a propagation direction (10) of the ultrasonic waves comprises a curved (11) inner surface (9) constituting a scattering element (6).

13. The system (1) according to any of the preceding claims, wherein the wall (7) of the treatment enclosure (2) opposite the ultrasonic transducer (5) and in a propagation direction (10) of the ultrasonic waves is parallel to the direction of the water stream (13).

14. The system (1) according to any of the preceding claims, wherein the at least one scattering element (6) is a conical, hemispherical, tetrahedral, quadrilateral pyramid, frustum or irregular structure.

15. Use of the system (1) according to any of the preceding claims for reducing and/or eliminating bacteria, such as legionella bacteria, in the water stream (13).
